# EUROPÄISCHE PATENTSCHRIFT

(11) **EP 3 262 456 B1**
(45) Veröffentlichungstag und Bekanntmachung des Hinweises auf die Patenterteilung: **28.09.2022**
(21) Anmeldenummer: 16702699.6
(22) Anmeldetag: 01.02.2016
(51) Int. Cl.: G02B 23/24, A61B 1/00, G02B 7/02, F16B 1/00

(54) **BAUEINHEIT EINES ENDOSKOPS MIT EINEM ROHR UMFASSEND FORMGEDÄCHTNISMATERIAL ZUR KLEMMFASSUNG OPTISCHER ELEMENTE**
ASSEMBLY OF AN ENDOSCOPE HAVING A TUBE COMPRISING SHAPE-MEMORY MATERIAL FOR THE CLAMP-MOUNTING OF OPTICAL ELEMENTS
UNITÉ STRUCTURALE D'UN ENDOSCOPE PRÉSENTANT UN TUBE COMPRENANT UN MATÉRIAU À MÉMOIRE DE FORME PERMETTANT DE MONTER PAR SERRAGE DES ÉLÉMENTS OPTIQUES

(30) Priorität: 25.02.2015 DE 102015203357
(43) Veröffentlichungstag der Anmeldung: 03.01.2018
(73) Patentinhaber: Olympus Winter&Ibe GmbH, 22045 Hamburg (DE)
(72) Erfinder: SCHÖLER, Uwe, 22955 Hoisdorf (DE)
(74) Vertreter: Seemann & Partner Patentanwälte mbB
(86) Internationale Anmeldenummer: PCT/EP2016/052060
(87) Internationale Veröffentlichungsnummer: WO 2016/134919

(56) Entgegenhaltungen:
- DE-A1- 3 007 307
- DE-A1- 19 732 991
- JP-A- S63 208 018
- US-A- 5 607 435
- US-A1- 2012 176 613

## Beschreibung

Die Erfindung betrifft eine optische Baueinheit eines Endoskops umfassend ein Objektivrohr und zumindest ein in dem Objektivrohr gehaltenes optisches Element. Ferner betrifft die Erfindung ein Endoskop umfassend eine optische Baueinheit, ein Verfahren zum Herstellen einer optischen Baueinheit sowie die Verwendung eines Formgedächtnismaterials.

Mit einem Endoskopobjektiv wird ein Untersuchungsgebiet vor einem distalen Ende des Endoskopschafts beobachtet. Gegenüber dem Außenraum ist das Endoskopobjektiv typischerweise mit einem Schutzfenster abgedichtet. Das Endoskopobjektiv bildet das Untersuchungsgebiet auf einen Bildsensor ab. Alternativ wird das Bild über Relaisoptiken bis in den proximalen Bereich des Endoskops projiziert. Dort befindet sich der Bildsensor oder ein Okular zur direkten Beobachtung des Untersuchungsgebiets. Bei flexiblen Endoskopen erfolgt die optische Abbildung auf ein flexibles Bündel optischer Fasern.

Das Endoskopobjektiv und die Relaisoptiken werden nachfolgend allgemein als optische Baueinheiten des Endoskops bezeichnet. Sie umfassen üblicherweise mehrere Linsen oder Linsengruppen, die in Längsrichtung des Endoskops hintereinander angeordnet und in einem Objektiv- oder Systemrohr gehalten sind.

Aus DE 10 2008 038 619 B3 geht ein Endoskopobjektiv mit drei Linsengruppen hervor, die mit Umfangsspiel in einem Objektivrohr aufgenommen sind. Das Objektivrohr umfasst einen innenliegenden Flansch, der in Längsaxialrichtung als Anschlag für die Linsengruppen dient. In radialer Richtung werden die Linsen fixiert, indem durch Bohrungen im Objektivrohr ein dünnflüssiger Klebstoff in den Spalt zwischen Linse bzw. Linsengruppe und Objektivrohr gegeben wird. Nach dem Aushärten des Klebstoffs liegt eine sichere Verbindung zwischen der Linsengruppe und dem Objektivrohr vor.

Ferner ist aus der US 2012/0176613 A1 eine Vorrichtung zur Aufnahme optischer Fasern sowie von Gradienten-Index-Linsen, wie sie für die Raman-Spektroskopie eingesetzt werden, bekannt. Es ist offenbart, eine Single-Mode-Faser in einem Edelstahlröhrchen aufzunehmen, dieses ist ebenso wie die am anderen Ende vorhandene Gradienten-Index-Linse in einem Nitinol-Röhrchen aufgenommen. Es wird Epoxidharz verwendet, um die genannten Komponenten zu fixieren.

Aus JPS 63-208018 A1 ist eine Linsenfassung bekannt, in der seitlich zwei Linsen eingebracht sind. Der Linsenhalter ist aus einer Formgedächtnislegierung hergestellt.

Aus US 5,607,435 A1 ist ein Endoskop bekannt, welches eine Linse umfasst, die in einem rohrförmigen Abschnitt aufgenommen ist. Um diesen rohrförmigen Abschnitt ist ein Sicherungsring gelegt, der aus einem Formgedächtnismaterial hergestellt ist.

Es ist eine Aufgabe der Erfindung, eine optische Baueinheit eines Endoskops, ein Endoskop umfassend eine optische Baueinheit, ein Verfahren zum Herstellen einer optischen Baueinheit sowie die Verwendung eines Formgedächtnismaterials anzugeben, wobei die optische Baueinheit effizienter herstellbar ist und gleichzeitig hohen optischen Anforderungen genügt.

Die Aufgabe wird gelöst durch eine optische Baueinheit eines Endoskops umfassend ein Objektivrohr und zumindest ein in dem Objektivrohr gehaltenes optisches Element, wobei die optische Baueinheit dadurch fortgebildet ist, dass das Objektivrohr zumindest einen als Klemmfassung für das optische Element wirkenden Bereich umfasst, wobei dieser als Klemmfassung wirkende Bereich des Objektivrohrs zumindest abschnittsweise aus einem Formgedächtnismaterial hergestellt ist, wobei ein Memory-Effekt des Formgedächtnismaterials von einer verformten Form in eine trainierte Form bei Überschreiten einer Memory-Temperatur eintritt und die trainierte Form des Objektivrohrs so gewählt ist, dass ein Klemmsitz zwischen dem zu haltenden optischen Element und dem als Klemmfassung wirkenden Bereich des Objektivrohrs bereitgestellt wird, wobei zumindest ein erstes optisches Element und ein zweites optisches Element vorhanden sind, wobei das erste optische Element in einem als Klemmfassung für das erste optische Element wirkenden ersten Bereich des Objektivrohrs und das zweite optische Element in einem als Klemmfassung für das zweite optische Element wirkenden zweiten Bereich des Objektivrohrs gehalten sind, und wobei das Objektivrohr zwischen dem ersten Bereich und dem zweiten Bereich einen verformbaren Übergangsbereich umfasst, der leichter verformbar als die sich benachbart anschließenden ersten und zweiten Bereiche des Objektivrohrs ist, und wobei der erste Bereich einen ersten Querschnitt und der zweite Bereich einen davon verschiedenen zweiten Querschnitt aufweist, wobei der erste und der zweite Querschnitt unterschiedlich in Größe oder Form sind und wobei der verformbare Übergangsbereich einen Übergang vom ersten Querschnitt auf den zweiten Querschnitt zwischen dem ersten und dem zweiten Bereich schafft, wobei als Übergangsbereich zumindest ein Federelement vorhanden ist.

Unter einem Objektivrohr wird im Kontext der vorliegenden Beschreibung stets auch ein Systemrohr verstanden.

Die Erfindung beruht auf der folgenden Erkenntnis: Die Toleranzen der Einzelkomponenten bei der Herstellung optischer Baueinheiten, beispielsweise Endoskopobjektiven oder Relaisoptiken, stößt an die Grenze des technisch Machbaren und ist immer noch zu groß, um die steigenden optischen Anforderungen, vor allem im Hinblick auf hochauflösende Bilderfassung (HD, 4K und nachfolgende Technologien), zu erfüllen. Ein Presssitz des optischen Elements in dem als Klemmfassung für das optische Element wirkenden Bereich des Objektivrohrs führt - was erfindungsgemäß erkannt wurde - zur Möglichkeit, diese Forderungen auf mechanischer Seite im Hinblick auf Toleranzen, Fertigungsgenauigkeit und Justagepräzision der optischen Elemente zu erfüllen. Die optischen Elemente, beispielsweise Linsen oder Linsengruppen, werden mit hoher Präzision auf der optischen Achse des optischen Systems zentriert. Dieser Effekt tritt ein, da das Formgedächtnismaterial in der Lage ist, einen gleichmäßig von allen Seiten wirkenden Anpressdruck auf das optische Element auszuüben.

Gemäß einer vorteilhaften Ausführungsform ist die optische Baueinheit dadurch fortgebildet, dass der als Klemmfassung wirkende Bereich des Objektivrohrs das optische Element entlang eines Umfangs des Objektivrohrs vollständig umschließt und ferner vollständig aus dem Formgedächtnismaterial hergestellt ist.

Der Umfang des Objektivrohrs wird in einer Ebene senkrecht zur optischen Achse des optischen Systems bzw. senkrecht zur Mittenlängsachse des Objektivrohrs betrachtet.

Eine vollflächige Anlage des optischen Elements entlang seines Umfangs an dem Objektivrohr ist nicht zwangsläufig vorgesehen. Es ist vorteilhaft, wenn das optische Element definiert an einzelnen Punkten, welche insbesondere gleichmäßig entlang des Umfangs des optischen Elements verteilt sind, in dem als Klemmfassung wirkenden Bereich des Objektivrohrs gehalten wird. Diese Art der Klemmhalterung erlaubt eine spielfreie, präzise und definierte Aufnahme des optischen Elements in der durch das Objektivrohr bereitgestellten Klemmhalterung.

Zumindest ein erstes optisches Element und ein zweites optisches Element sind vorhanden, wobei das erste optische Element in einem als Klemmfassung für das erste optische Element wirkenden ersten Bereich des Objektivrohrs und das zweite optische Element in einem als Klemmfassung für das zweite optische Element wirkenden zweiten Bereich des Objektivrohrs gehalten sind, und wobei das Objektivrohr zwischen dem ersten Bereich und dem zweiten Bereich einen verformbaren Übergangsbereich umfasst.

Im Kontext der vorliegenden Beschreibung bedeutet "verformbarer Übergangsbereich", dass dieser Übergangsbereich plastisch oder elastisch leichter verformbar bzw. mechanisch weicher ist als die benachbart anschließenden Bereiche bzw. Abschnitte des Objektivrohrs. Als Übergangsbereich sind erfindungsgemäss Federelemente vorgesehen. Ferner ist es möglich, den Übergangsbereich aus einem Metall oder aus Kunststoff herzustellen.

Vorteilhaft wird die Möglichkeit geschaffen, unterschiedlich große und unterschiedlich geformte optische Elemente spielfrei, fest und sicher in dem Objektivrohr aufzunehmen. Das Formgedächtnismaterial ist lokal, d.h. in jedem einzelnen Bereich, in der Lage, sich optimal an das dort aufgenommene bzw. gehaltene optische Element anzuschmiegen und dieses sicher zu halten.

Es ist vorgesehen, dass der erste Bereich einen ersten Querschnitt und der zweite Bereich einen davon verschiedenen zweiten Querschnitt aufweist, wobei der erste und der zweite Querschnitt unterschiedlich in Größe oder Form sind, und wobei der verformbare Übergangsbereich einen Übergang vom ersten Querschnitt auf den zweiten Querschnittzwischen dem ersten und dem zweiten Bereich, schafft.

Unterschiedliche Formen der optischen Elemente, welche einen unterschiedlichen Querschnitt, sei es in Größe oder Form, erfordern, lassen sich in der optischen Baueinheit gemäß der genannten Ausführungsform aufnehmen. Gleiches gilt beispielsweise für verschieden große Linsen, welche unterschiedlich große Durchmesser aufweisen. Insbesondere sind die einzelnen Bereiche des Objektivrohrs so zueinander angeordnet, dass die in ihnen aufgenommenen optischen Elemente präzise auf einer gemeinsamen optischen Achse gehalten werden. Es ist ferner vorgesehen, dass in unterschiedlichen Bereichen unterschiedliche Klemmkräfte auf die dort gehaltenen optischen Elemente ausgeübt werden.

Gemäß einer weiteren Ausführungsform ist vorgesehen, dass der als Klemmfassung wirkende zumindest eine Bereich des Objektivrohrs eine trainierte Form hat, die im Querschnitt des Objektivrohrs betrachtet polygonförmig ist, wobei insbesondere eine verformte Form des Objektivrohrs zumindest in dem als Klemmfassung wirkenden Bereich kreisrund ist.

Im Kontext der vorliegenden Beschreibung ist eine "trainierte Form" diejenige Form des Formgedächtnismaterials, an die es sich "erinnert", wenn es auf eine Temperatur oberhalb der Memory-Temperatur erwärmt wird. Als "verformte Form" wird diejenige Form bezeichnet, in die das Material durch Verformung unterhalb einer kritischen Temperatur gebracht wurde, so dass kein Trainieren dieser Form stattfindet. Der Formgedächtniseffekt oder Memory-Effekt tritt also von der verformten Form in die trainierte Form ein, wenn die Memory-Temperatur überschritten wird.

Die trainierte Form ist insbesondere so gewählt, dass ein Klemmsitz zwischen dem optischen Element und dem als Klemmfassung wirkenden Bereich des Objektivrohrs bereitgestellt wird. Die trainierte Form wird also mit anderen Worten so gewählt, dass eine erzeugte Klemmkraft für das aufzunehmende optische Element weder zu hoch noch zu gering ist.

Bevorzugt kommt das optische Element jeweils in der Mitte einer Seite der genannten Vielecke zur Anlage. Eine Dreiecksform des Objektivrohrs, zumindest in dem als Klemmfassung für das optische Element wirkenden Bereich, ist besonders vorteilhaft, da eine Dreipunktaufnahme die, geometrisch gesehen, stabilste Aufnahme des optischen Elements darstellt. Ein sechseckiges Profil hat den Vorteil, dass zur Justage auf der optischen Achse Querstreben eingesetzt werden können, die nach erfolgtem Klemmvorgang wieder entfernt werden.

Gemäß einer weiteren Ausführungsform ist die optische Baueinheit dadurch fortgebildet, dass das Formgedächtnismaterial eine Formgedächtnislegierung und/oder ein Formgedächtnispolymer ist.

Eine Formgedächtnislegierung oder ein Memory-Metall ist vielfach eine Nickel-Titan-Legierung, beispielsweise ein unter dem Namen "Nitinol" bekanntes Material.

Bei dem optischen Element handelt es sich insbesondere um eine Linse. Ferner handelt es sich bevorzugt um miteinander verklebte Linsen, die eine Linsengruppe bilden. Die optischen Elemente der optischen Baueinheit werden ferner bevorzugt ohne weitere Haltemaßnahmen, wie beispielsweise eine Verklebung, Verschraubung oder dgl., in dem Objektivrohr gehalten. Die oben genannten Aspekte betreffen vorteilhaft alle Ausführungsformen.

Die Aufgabe wird ferner gelöst durch ein Endoskop umfassend eine optische Baueinheit nach einem oder mehreren der genannten Ausführungsformen als Endoskopobjektiv oder Relaisoptik, wobei insbesondere das optische Element eine Linse oder eine Linsengruppe ist.

Das Endoskopobjektiv gemäß Aspekten der Erfindung ist für die hochauflösende Bilderfassung geeignet, da im Hinblick auf Toleranzen der Einzelkomponenten der optischen Baueinheit die vorhandenen technischen Beschränkungen vorteilhaft überwunden werden.

Ferner wird die Aufgabe gelöst durch ein Verfahren zum Herstellen einer optischen Baueinheit nach einem oder mehreren der genannten Ausführungsformen, wobei das Verfahren durch die folgenden Schritte fortgebildet ist:
- Bereitstellen eines Objektivrohrs, welches zumindest einen als Klemmfassung für das optische Element wirkenden Bereich umfasst, der zumindest abschnittsweise aus einem Formgedächtnismaterial hergestellt ist,
- Einsetzen zumindest eines optischen Elements in den als Klemmfassung wirkenden Bereich des Objektivrohrs,
- Erwärmen des zumindest einen als Klemmfassung wirkenden Bereich des Objektivrohrs auf eine Temperatur oberhalb der Memory-Temperatur des verwendeten Formgedächtnismaterials.

Ferner wird die Aufgabe gelöst durch die Verwendung eines Formgedächtnismaterials zum Herstellen eines Objektivrohrs einer optischen Baueinheit nach einem oder mehreren der genannten Ausführungsformen. Insbesondere wird ein Formgedächtnismetall oder eine Formgedächtnislegierung, bevorzugt Nitinol, verwendet.

Auf das Verfahren und die Verwendung treffen gleiche oder ähnliche Vorteile zu, wie bereits im Hinblick auf die optische Baueinheit eines Endoskops erwähnt wurden, weshalb auf eine erneute Nennung verzichtet wird.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: ein Endoskop in schematischer, vereinfachter und perspektivischer Ansicht,
- Fig. 2: eine optische Baueinheit eines Endoskops in einem schematisch vereinfachten Längsschnitt,
- Fig. 3a, 3b und 4a, 4b: schematisch vereinfachte Querschnittsansichten durch ein Objektivrohr in verformter Form und in trainierter Form und
- Fig. 5: eine schematisch vereinfachte und perspektivische Ansicht eines Objektivrohrs.

In den Zeichnungen sind jeweils gleiche oder gleichartige Elemente und/oder Teile mit denselben Bezugsziffern versehen, so dass von einer erneuten Vorstellung jeweils abgesehen wird.

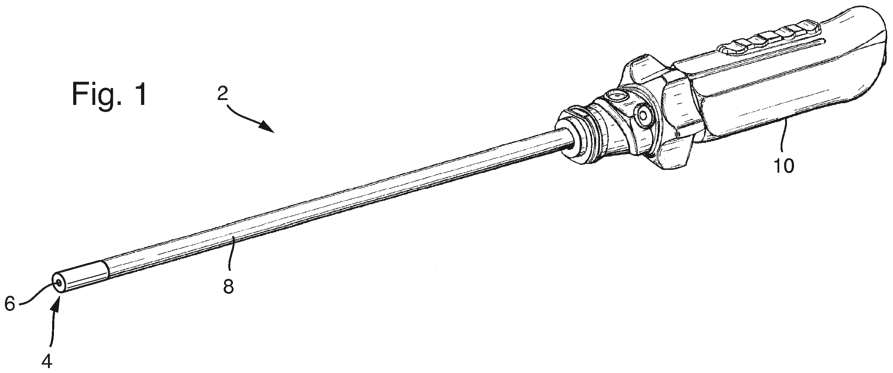

Fig. 1 zeigt in vereinfachter, schematischer und perspektivischer Darstellung ein Endoskop 2, beispielhaft ein starres Endoskop, an dessen distalem Ende 4 ein Endoskopobjektiv 6 vorhanden ist, welches hinter einer nicht dargestellten Schutzscheibe angeordnet ist. In einem Schaft 8 des Endoskops 2 befinden sich ferner je nach Typ des Endoskops 2 nicht dargestellte Relaisoptiken. Das Endoskopobjektiv 6 und diese Relaisoptiken bilden optische Einheiten des Endoskops 2. Ein proximales Ende des Endoskops 2 umfasst einen Handgriff 10.

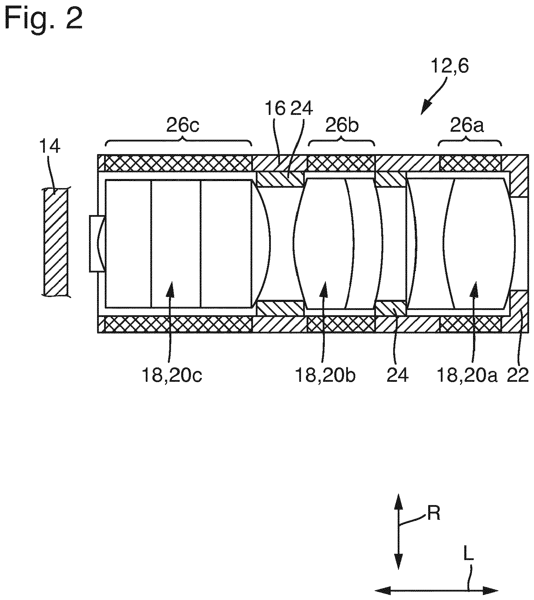

Fig. 2 zeigt in einer vereinfachten und schematischen Längsschnittansicht eine optische Baueinheit 12 eines Endoskops 2, beispielhaft ein Endoskopobjektiv 6. Dieses ist hinter der Schutzscheibe 14 am distalen Ende 4 des Schafts 6 des Endoskops 2 angeordnet. Die optische Baueinheit 12 umfasst ein Objektivrohr 16 und zumindest ein in dem Objektivrohr 16 gehaltenes optisches Element 18, beispielhaft drei optische Elemente 18, nämlich eine proximale Linsengruppe 20a, eine zentrale Linsengruppe 20b und eine distale Linsengruppe 20c. Lediglich beispielhaft sind Linsengruppen 20a, 20b, 20c dargestellt, welche jeweils aus mehreren einzelnen Linsen zusammengesetzt sind. Bei einem optischen Element handelt es sich ebenfalls um einzelne Listen, Prismen oder dgl.

Die optischen Elemente 18 sind in dem Objektivrohr 16 durch einen Endanschlag 22 am proximalen Ende des Objektivrohrs 16 sowie durch zwischen den Linsengruppen 20a, 20b, 20c vorhandene Blendrohre 24, die ebenfalls als Anschlag wirken, gehalten. In radialer Richtung R, die senkrecht zu der Längsaxialrichtung L der optischen Baueinheit 12 verläuft, sind die optischen Elemente 18 durch zumindest einen als Klemmfassung für die optischen Elemente 18 wirkenden Bereich 26a, 26b, 26c gehalten.

Dieser als Klemmfassung wirkende Bereich 26a, 26b, 26c des Objektivrohrs 16 ist zumindest abschnittsweise aus einem Formgedächtnismaterial hergestellt. Als Formgedächtnismaterial ist beispielsweise eine Formgedächtnislegierung oder ein Formgedächtnispolymer vorgesehen. Bevorzugt kommt eine Formgedächtnislegierung oder ein Formgedächtnismetall, wie beispielsweise Nitinol, zum Einsatz.

Der als Klemmfassung wirkende Bereich 26a, 26b, 26c des Objektivrohrs 16 umschließt das optische Element 18 entlang eines Umfangs des Objektivrohrs 16 vollständig. Im dargestellten Ausführungsbeispiel sind ein erster als Klemmfassung wirkender Bereich 26a, ein zweiter als Klemmfassung wirkender Bereich 26b und ein dritter als Klemmfassung wirkender Bereich 26c vorgesehen. Die Bereiche 26a, 26b, 26c haben jeweils die Form eines Hohlzylinders und umschließen das zugehörige optische Element in einer Ebene vollständig. Diese Ebene liegt senkrecht zur Längsaxialrichtung L, welche parallel zur optischen Achse des optischen Systems orientiert ist. Die Bereiche 26a, 26b, 26c des Objektivrohrs 16 sind bevorzugt vollständig aus einem Formgedächtnismaterial hergestellt.

Im dargestellten Ausführungsbeispiel umfasst das Objektivrohr 16 mehrere optische Elemente 18, nämlich die Linsengruppen 20a, 20b, 20c. Beispielhaft umschließt der erste Bereich 26a die proximale Linsengruppe 20a als erstes optisches Element 18, der zweite Bereich 26b die zentrale Linsengruppe 20b als zweites optisches Element 18 und der dritte Bereich 26c die distale Linsengruppe 20c als drittes optisches Element 18. Die zwischen der proximalen und der distalen Linsengruppe 20a, 20c vorhandene zentrale Linsengruppe 20b ist alternativ über die Blendrohre 24 gehalten.

Fig. 2 zeigt das Objektivrohr 16 in verformter Form, in welcher das Objektivrohr 16 im Querschnitt kreisrund ist. Durch eine anschließende Temperaturbehandlung oberhalb einer Memory-Temperatur verändert sich das Objektivrohr 16, genauer dessen Bereiche 26a, 26b, 26c in eine trainierte Form.

Im Kontext der vorliegenden Beschreibung ist unter einer trainierten Form diejenige Form des Formgedächtnismaterials zu verstehen, in die es bei einer entsprechend hohen Temperatur oberhalb der Memory-Temperatur gebracht wird. Als verformte Form wird die Form bezeichnet, in die das Material durch Verformung unterhalb einer kritischen Temperatur gebracht wird, so dass kein Trainieren dieser Form stattfindet. Der Memory-Effekt tritt von der verformten Form in die trainierte Form bei Überschreiten der Memory-Temperatur ein.

Die trainierte Form des Objektivrohrs 16 ist so gewählt, dass ein Klemmsitz zwischen dem zu haltenden optischen Element 18 und dem als Klemmfassung wirkenden Bereich 26a, 26b, 26c des Objektivrohrs 16 bereitgestellt wird. Die Geometrie und Abmessungen der trainierten Form stellen sicher, dass die optischen Elemente 18 mit einer adäquaten, also weder zu hohen noch zu geringen, Klemmkraft gehalten werden.

Abweichend von der Darstellung in Fig. 2 ist das Objektivrohr 16 ferner dazu vorgesehen, optische Elemente 18 verschiedenen Querschnitts, insbesondere optische Elemente 18 mit unterschiedlichem Durchmesser aufzunehmen. Hierzu ist es mit anderen Worten notwendig, dass die Bereiche 26a, 26b, 26c in dem Zustand, in dem diese den Klemmsitz für das optische Element 18 bereitstellen, ebenfalls unterschiedliche Querschnitte, insbesondere unterschiedliche Durchmesser, aufweisen. Zu diesem Zweck befinden sich zwischen den Bereichen 26a, 26b, 26c Übergangsbereiche. Die Übergangsbereiche sind verformbar, beispielsweise plastisch und/oder elastisch verformbar. Erfindungsgemäss sind als verformbarer Übergangsbereich Federelemente vorgesehen.

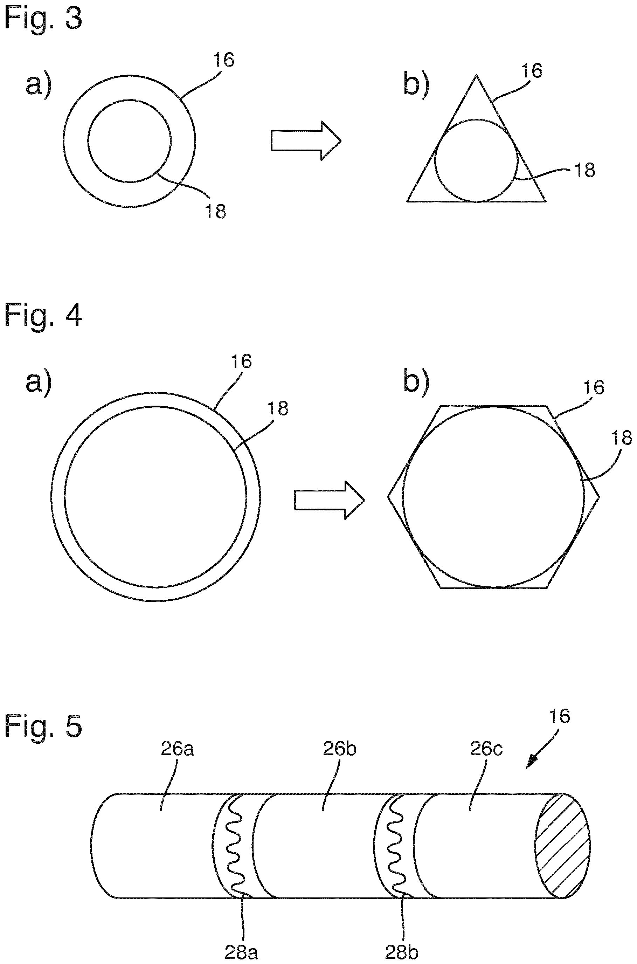

Fig. 3 zeigt in einer schematisch vereinfachten Querschnittsansicht ein Objektivrohr 16 in seiner verformten Form (Fig. 3a) und in seiner trainierten Form (Fig. 3b). Beispielhaft weist das Objektivrohr 16, genauer dessen Bereiche 26a, 26b, 26c eine trainierte Form auf, die, im Querschnitt des Objektivrohrs 16 betrachtet, dreieckig ist.

In der verformten Form wird das optische Element 18 in das Objektivrohr 16 eingesetzt. Anschließend werden die Bereiche 26a, 26b, 26c bevorzugt das gesamte Objektivrohr 16 einschließlich der optischen Elemente 18, auf eine Temperatur oberhalb der Memory-Temperatur des für die Bereiche 26a, 26b, 26c verwendeten Formgedächtnismaterials gebracht. Das Formgedächtnismaterial kehrt in seine trainierte Form zurück (Fig. 3b). Die Wandung des Objektivrohrs 16 kommt zur Anlage mit dem optischen Element 18, so dass ein Klemmsitz für das optische Element 18 bewirkt wird. Gemäß dem in Fig. 3 dargestellten Ausführungsbeispiel wird das optische Element 18 an drei Punkten entlang seines Umfangs gehalten. So wird ein spielfreier und daher besonders präziser und sicherer Sitz des optischen Elements 18 in dem Objektivrohr 16 bewirkt. Das optische Element 18 kommt entlang seines Umfangs jeweils in der Mitte einer jeden Seite des im Querschnitt betrachteten dreieckigen Objektivrohrs 16 zur Anlage.

Fig. 4 zeigt einen weiteren schematisch vereinfachten Querschnitt durch ein Objektivrohr 16 in seiner verformten Form (Fig. 4a) und in seiner trainierten Form (Fig. 4b). In der trainierten Form ist das Objektivrohr 16 gemäß diesem Ausführungsbeispiel im Querschnitt betrachtet sechseckig. Erneut kommt das optische Element 18 entlang seines Umfangs mittig an einer jeweiligen Seite dieses Sechsecks zur Anlage. Ein sechseckiger Querschnitt hat den Vorteil, das zur Justage der Lage des optischen Elements 18 Querstreben in das Objektivrohr 16, genauer in dessen Bereiche 26a, 26b, 26c eingesetzt werden können, welche nach Realisierung des Klemmsitzes des optischen Elements 18 in dem Objektivrohr 16 wieder entfernt werden.

Fig. 5 zeigt in einer schematischen, vereinfachten und perspektivischen Querschnittsansicht ein Objektivrohr 16, umfassend drei Bereiche 26a, 26b, 26c, zwischen denen zwei Übergangsbereiche 28a, 28b vorhanden sind. Es handelt sich bei dem ersten Übergangsbereich 26a, welcher zwischen dem ersten Bereich 26a und dem zweiten Bereich 26b angeordnet ist, um Federelemente. Diese sind in der Lage, sich elastisch zu verformen und so unterschiedliche Querschnitte des ersten und des zweiten Bereichs 26a, 26b, insbesondere unterschiedliche Durchmesser dieser Bereiche 26a, 26b, auszugleichen. Gleiches gilt für den zweiten Übergangsbereich 28b, der zwischen dem zweiten Bereich 26b und dem dritten Bereich 26c angeordnet ist.

Zum Herstellen einer optischen Baueinheit 12, beispielsweise eines Endoskopobjektivs 6, wird gemäß einem Ausführungsbeispiel wie folgt verfahren: Zunächst wird ein Objektivrohr 16 bereitgestellt, welches zumindest einen als Klemmfassung für das optische Element 18 wirkenden Bereich 26a, 26b, 26c umfasst. Dieser Bereich 26a, 26b, 26c ist, zumindest abschnittsweise, aus einem Formgedächtnismaterial, beispielsweise einem Formgedächtnismetall wie Nitinol, hergestellt. Insbesondere ist der Bereich 26a, 26b, 26c vollständig aus einem Formgedächtnismaterial hergestellt. Anschließend wird zumindest ein optisches Element 18, beispielsweise eine Linsengruppe 20a, 20b, 20c, insbesondere mehrere optische Elemente 18, wie beispielsweise die drei Linsengruppen 20a, 20b, 20c, in das Objektivrohr 16 eingesetzt. Durch anschließendes Erwärmen zumindest der Bereiche 26a, 26b, 26c, insbesondere des gesamten Objektivrohrs 16, einschließlich der eingesetzten optischen Elemente 18, auf eine Temperatur oberhalb der Memory-Temperatur des verwendeten Formgedächtnismaterials, wird ein Klemmsitz zwischen dem als Klemmfassung wirkenden Bereich 26a, 26b, 26c und dem optischen Element 18 hergestellt.

Mithilfe des Objektivrohrs 16 gemäß Aspekten der Erfindung, welches insbesondere unter Verwendung eines Formgedächtnismaterials hergestellt ist, sind das oder die optischen Elemente 18 exakt in dem Objektivrohr 16 aufnehmbar. Vorteilhaft entfällt die Notwendigkeit, weitere Maßnahmen zum Fixieren der optischen Elemente 18, wie beispielsweise Klebstoff, vorzusehen. Die optischen Elemente 18 sind derart genau in dem Objektivrohr 16 aufnehmbar, das hohe optische Anforderungen an die Justage des optischen Systems erfüllt werden. Insbesondere ist die bereitgestellte optische Einheit 12, beispielsweise das Endoskopobjektiv 6, zur Erfassung hoch auflösender Bildinformationen (HD, ultra HD, 4K und höher) geeignet.

### Bezugszeichenliste

- 2: Endoskop
- 4: distales Ende
- 6: Endoskopobjektiv
- 8: Schaft
- 10: Handgriff
- 12: optische Baueinheit
- 14: Schutzscheibe
- 16: Objektivrohr
- 18: optisches Element
- 20a: proximale Linsengruppe
- 20b: zentrale Linsengruppe
- 20c: distale Linsengruppe
- 22: Endanschlag
- 24: Blendrohr
- 26a: erster Bereich
- 26b: zweiter Bereich
- 26c: dritter Bereich
- 28a: erster Übergangsbereich
- 28b: zweiter Übergangsbereich

- R: radiale Richtung
- L: Längsaxialrichtung

## Patentansprüche

1. Optische Baueinheit (12) eines Endoskops (2) umfassend ein Objektivrohr (16) und zumindest ein in dem Objektivrohr (16) gehaltenes optisches Element (18), wobei das Objektivrohr (16) zumindest einen als Klemmfassung für das optische Element (18) wirkenden Bereich (26a, 26b, 26c) umfasst, wobei dieser als Klemmfassung wirkende Bereich (26a, 26b, 26c) des Objektivrohrs (16) zumindest abschnittsweise aus einem Formgedächtnismaterial hergestellt ist, wobei ein Memory-Effekt des Formgedächtnismaterials von einer verformten Form in eine trainierte Form bei Überschreiten einer Memory-Temperatur eintritt und die trainierte Form des Objektivrohrs (16) so gewählt ist, dass ein Klemmsitz zwischen dem zu haltenden optischen Element (18) und dem als Klemmfassung wirkenden Bereich (26a, 26b, 26c) des Objektivrohrs (16) bereitgestellt wird, wobei zumindest ein erstes optisches Element (18, 20a) und ein zweites optisches Element (18, 20b) vorhanden sind, wobei das erste optische Element (18, 20a) in einem als Klemmfassung für das erste optische Element (18, 20a) wirkenden ersten Bereich (26a) des Objektivrohrs (16) und das zweite optische Element (18, 20b) in einem als Klemmfassung für das zweite optische Element (18, 20b) wirkenden zweiten Bereich (26b) des Objektivrohrs (16) gehalten sind, wobei das Objektivrohr (16) zwischen dem ersten Bereich (26a) und dem zweiten Bereich (26b) einen verformbaren Übergangsbereich (28a, 28b) umfasst, der leichter verformbar als die sich benachbart anschließenden ersten und zweiten Bereiche (26a, 26b) des Objektivrohrs (16) ist, und wobei der erste Bereich (26a) einen ersten Querschnitt und der zweite Bereich (26b) einen davon verschiedenen zweiten Querschnitt aufweist, wobei der erste und der zweite Querschnitt unterschiedlich in Größe oder Form sind und wobei der verformbare Übergangsbereich (28a, 28b) einen Übergang vom ersten Querschnitt auf den zweiten Querschnitt zwischen dem ersten und dem zweiten Bereich (26a, 26b) schafft, und wobei als Übergangsbereich (28a, 28b) zumindest ein Federelement vorhanden ist.

2. Optische Baueinheit (12) nach Anspruch 1, **dadurch gekennzeichnet, dass** der als Klemmfassung wirkende Bereich (26a, 26b, 26c) des Objektivrohrs (16) das optische Element (18) entlang eines Umfangs des Objektivrohrs (16) vollständig umschließt und ferner vollständig aus dem Formgedächtnismaterial hergestellt ist.

3. Optische Baueinheit (12) nach Anspruch 1 oder 2, **dadurch gekennzeichnet, dass** der als Klemmfassung wirkende zumindest eine Bereich (26a, 26b, 26c) des Objektivrohrs (16) eine trainierte Form hat, die im Querschnitt des Objektivrohrs (16) betrachtet polygonförmig ist, wobei eine verformte Form des Objektivrohrs (16) zumindest in dem als Klemmfassung wirkenden Bereich (26a, 26b, 26c) kreisrund ist.

4. Optische Baueinheit (12) nach einem der Ansprüche 1 bis 3, **dadurch gekennzeichnet, dass** das Formgedächtnismaterial eine Formgedächtnislegierung und/oder ein Formgedächtnispolymer ist.

5. Endoskop (2) umfassend eine optische Baueinheit (12) nach einem der Ansprüche 1 bis 4 als Endoskopobjektiv (6) oder Relaisoptik.

6. Verfahren zum Herstellen einer optischen Baueinheit (12) nach einem der Ansprüche 1 bis 5, **gekennzeichnet durch** die folgenden Schritte:
- Bereitstellen eines Objektivrohrs (16), welches zumindest einen als Klemmfassung für das optische Element (18) wirkenden Bereich (26a, 26b, 26c) umfasst, der zumindest abschnittsweise aus einem Formgedächtnismaterial hergestellt ist,
- Einsetzen zumindest eines optischen Elements (18) in den als Klemmfassung wirkenden Bereich (26a, 26b, 26c) des Objektivrohrs (16),
- Erwärmen des zumindest einen als Klemmfassung wirkenden Bereichs (26a, 26b, 26c) des Objektivrohrs (16) auf eine Temperatur oberhalb der Memory-Temperatur des verwendeten Formgedächtnismaterials.

7. Verwendung eines Formgedächtnismaterials zum Herstellen eines Objektivrohrs (16) einer optischen Baueinheit (12) nach einem der Ansprüche 1 bis 4 eines Endoskops (2) nach Anspruch 5.

## Claims

1. An optical assembly (12) of an endoscope (2) comprising an objective tube (16) and at least one optical element (18) held in the objective tube (16), wherein the objective tube (16) comprises at least one region (26a, 26b, 26c) functioning as a clamping mount for the optical element (18), wherein this region (26a, 26b, 26c) of the objective tube (16) functioning as a clamping mount is manufactured at least sectionally from a shape-memory material, wherein a memory effect of the shape-memory material from a deformed shape to a trained shape occurs when a memory temperature is exceeded and the trained shape of the objective tube (16) is selected in that there is a clamping mount between the optical element (18) to be held and the region (26a, 26b, 26c) of the objective tube (16) functioning as a clamping mount, wherein at least one first optical element (18, 20a) and one second optical element (18, 20b) are available, wherein the first optical element (18, 20a) is held in a first region (26a) of the objective tube (16) functioning as a clamping mount for the first optical element (18, 20a) and the second optical element (18, 20b) is held in a second region (20b) of the objective tube (16) functioning as a clamping mount for the second optical element (18, 20b), wherein the objective tube (16) comprises a deformable transition region (28a, 28b) between the first region (26a) and the second region (26b), which is easier deformable than the neighboring adjacent first and second regions (26a, 26b) of the objective tube (16) and wherein the first region (26a) has a first cross-section and the second region (26b) has a second cross-section different therefrom, wherein the first and the second cross-section are different in size and shape and wherein the deformable transition region (28a, 28b) creates a transition from the first cross-section to the second cross-section between the first and second region (26a, 26b) and wherein at least one spring element is provided as the transition region (28a, 28b).

2. The optical assembly (12) according to claim 1, **characterized in that** the region (26a, 26b, 26c) of the objective tube (16) acting as a clamping mount fully encloses the optical element (18) along a circumference of the objective tube (16) and moreover is made completely from the shape-memory material.

3. The optical assembly (12) according to claim 1 or 2, **characterized in that** the at least one region (26a, 26b, 26c) of the objective tube (16) functioning as the clamping mount has a trained shape which is polygonal viewed in the cross-section of the objective tube (16), wherein a deformed shape of the objective tube (16) is circular, at least in the region (26a, 26b, 26c) functioning as the clamping mount.

4. The optical assembly (12) according to one of claims 1 to 3 **characterized in that** the shape-memory material is a shape-memory alloy and/or a shape-memory polymer.

5. An endoscope (2) comprising an optical assembly (12) according to one of claims 1 to 4 as an endoscope objective (6) or relay lens.

6. A method for producing an optical assembly (12) according to one of claims 1 to 5, **characterized by** the following steps:
- Providing an objective tube (16) that at least comprises a region (26a, 26b, 26c) which functions as a clamping mount for the optical element (18) and is at least sectionally manufactured from a shape-memory material,
- Inserting at least one optical element (18) into the region (26a, 26b, 26c) of the objective tube (16) functioning as a clamping mount,
- Heating the at least one region (26a, 26b, 26c) of the objective tube (16) functioning as a clamping mount to a temperature above the memory temperature of the used shape-memory material.

7. A use of a shape-memory material for manufacturing an objective tube (16) of an optical assembly (12) according to one of claims 1 to 4 of an endoscope (2) according to claim 5.

## Revendications

1. Unité optique (12) d'un endoscope (2) comprenant un tube d'objectif (16) et au moins un élément optique (18) maintenu dans le tube d'objectif (16), le tube d'objectif (16) comprenant au moins une zone (26a, 26b, 26c) agissant en tant que monture de serrage pour l'élément optique (18), cette zone (26a, 26b, 26c) du tube d'objectif (16) qui agit en tant que monture de serrage étant fabriquée au moins par sections à partir d'un matériau à mémoire de forme, un effet de mémoire du matériau à mémoire de forme se produisant d'une forme déformée à une forme entraînée en cas de dépassement d'une température de mémoire et la forme entraînée du tube d'objectif (16) étant choisie de telle sorte qu'un ajustement serré est prévu entre l'élément optique (18) à maintenir et la zone (26a, 26b, 26c) du tube d'objectif (16) agissant en tant que monture de serrage, au moins un premier élément optique (18, 20a) et un deuxième élément optique (18, 20b) étant présents, le premier élément optique (18, 20a) étant maintenu dans une première zone (26a) du tube d'objectif (16) agissant en tant que monture de serrage pour le premier élément optique (18, 20a) et le deuxième élément optique (18, 20b) étant maintenu dans une deuxième zone (26b) du tube d'objectif (16) agissant en tant que monture de serrage pour le deuxième élément optique (18, 20b), dans lequel
le tube d'objectif (16) comprend, entre la première zone (26a) et la deuxième zone (26b), une zone de transition déformable (28a, 28b) qui est plus facilement déformable que les première et deuxième zones adjacentes (26a, 26b) du tube d'objectif (16), et dans lequel la première zone (26a) présente une première section transversale et la deuxième zone (26b) une deuxième section transversale différente de la première, la première et la deuxième sections transversales étant différentes en taille ou en forme et la zone de transition déformable (28a, 28b) créant une transition de la première section transversale à la deuxième section transversale entre la première et la deuxième zones (26a, 26b), et au moins un élément à ressort étant présent en tant que zone de transition (28a, 28b).

2. Unité optique (12) selon la revendication 1, **caractérisée en ce que** la zone (26a, 26b, 26c) du tube d'objectif (16) agissant en tant que monture de serrage entoure complètement l'élément optique (18) le long d'une circonférence du tube d'objectif (16) et est en outre entièrement fabriquée à partir du matériau à mémoire de forme.

3. Unité optique (12) selon la revendication 1 ou la revendication 2, **caractérisée en ce que** ladite au moins une zone (26a, 26b, 26c) du tube d'objectif (16) agissant en tant que monture de serrage a une forme entraînée qui, vue en coupe transversale du tube d'objectif (16), est de forme polygonale, une forme déformée du tube d'objectif (16) étant circulaire au moins dans la zone (26a, 26b, 26c) agissant en tant que monture de serrage.

4. Unité optique (12) selon l'une des revendications 1 à 3, **caractérisée en ce que** le matériau à mémoire de forme est un alliage à mémoire de forme et/ou un polymère à mémoire de forme.

5. Endoscope (2) comprenant un unité optique (12) selon l'une quelconque des revendications 1 à 4 en tant qu'objectif d'endoscope (6) ou qu'optique de relais.

6. Procédé de fabrication d'un unité optique (12) selon l'une des revendications 1 à 5, **caractérisé par** les étapes suivantes :
- Fourniture d'un tube d'objectif (16) comprenant au moins une zone (26a, 26b, 26c) agissant en tant que monture de serrage pour l'élément optique (18), réalisée au moins en partie en un matériau à mémoire de forme,
- Insertion d'au moins un élément optique (18) dans la zone (26a, 26b, 26c) du tube d'objectif (16) agissant en tant que monture de serrage,
- Chauffage de ladite au moins une zone (26a, 26b, 26c) du tube d'objectif (16) agissant en tant que monture de serrage à une température supérieure à la température de mémoire du matériau à mémoire de forme utilisé.

7. Utilisation d'un matériau à mémoire de forme pour la fabrication d'un tube d'objectif (16) d'un unité optique (12) selon l'une des revendications 1 à 4 d'un endoscope (2) selon la revendication 5.
